# EUROPÄISCHE PATENTSCHRIFT

(11) **EP 0 866 731 B1**
(45) Veröffentlichungstag und Bekanntmachung des Hinweises auf die Patenterteilung: **27.03.2002**
(21) Anmeldenummer: 96931803.9
(22) Anmeldetag: 13.09.1996
(51) Int. Cl.: B01J 23/78, B01J 37/08, B01J 37/32, C07C 5/333, C07C 15/46

(54) **VERFAHREN ZUR HERSTELLUNG VON KATALYSATOREN FÜR SELEKTIVE DEHYDRIERUNGEN SOWIE DADURCH HERGESTELLTE KATALYSATOREN**
METHOD OF PRODUCING SELECTIVE-DEHYDROGENATION CATALYSTS, AND CATALYSTS PRODUCED IN THIS WAY
PROCEDE DE PRODUCTION DE CATALYSEURS POUR LA DESHYDROGENATION SELECTIVE ET CATALYSEURS OBTENUS A L'AIDE DUDIT PROCEDE

(30) Priorität: 23.09.1995 DE 19535416
(43) Veröffentlichungstag der Anmeldung: 30.09.1998
(73) Patentinhaber: BASF AKTIENGESELLSCHAFT, 67056 Ludwigshafen (DE)
(72) Erfinder: BAIER, Michael, D-81241 München (DE); RIEKER, Christopher, William, D-67454 Hassloch (DE); HOFSTADT, Otto, D-67122 Altrip (DE); BÜCHELE, Wolfgang, D-67063 Ludwigshafen (DE); PÖPEL, Wolfgang, Jürgen, D-64297 Darmstadt (DE); PETERSEN, Hermann, D-67269 Grünstadt (DE)
(86) Internationale Anmeldenummer: EP9604028
(87) Internationale Veröffentlichungsnummer: WO9710898

(56) Entgegenhaltungen:
- EP-A- 0 177 832
- EP-A- 0 296 285
- WO-A-94/25154
- WO-A-96/18594
- DE-A- 2 534 467
- US-A- 4 144 197

## Beschreibung

Die Erfindung betrifft ein verbessertes Verfahren zur Herstellung von Eisen und Kalium und bevorzugt Cer und gegebenenfalls weitere Elemente enthaltenden Katalysatoren, die sich zur Dehydrierung von Kohlenwasserstoffen zu den entsprechenden, olefinisch ungesättigten Kohlenwasserstoffen, insbesondere zur Dehydrierung von Ethylbenzol zu Styrol eignen.

Solche Katalysatoren und deren Herstellung sind beispielsweise in der US-PS 3 904 552 (1), den europäischen Patentschriften 177 832 (2), 181 999 (3), 195 252 (4), 296 285 (5), 297 685 (6), 297 657 (7) und 502 510 (8) und den deutschen Offenlegungsschriften 28 15 812 (9), 28 15 874 (10), 36 43 382 (11) und 38 21 431 (12) beschrieben.

Außer Eisen und Kalium enthalten die bekannten Katalysatoren i.a. sog. Promotoren, also Elemente, die mindestens nach der Herstellung i.a. in Form ihrer Oxide vorliegen. Als Promotoren werden z.B. Mo, Ce (1), W (4), Mg (2, 3), V, Cr (9), Ti (8) und andere Elemente beschrieben. Bei der Herstellung werden diese Bestandteile zusammen mit den Eisen und Kalium liefernden Verbindungen in Form von Oxiden oder zersetzbaren Verbindungen zu pastosen Massen verarbeitet, die die Bestandteile des künftigen Katalysators in Form sogenannter "precursor" enthalten und beim nachfolgenden Erhitzen (Calcinieren) Oxide bilden. Eventuell werden noch Hilfsmittel, z.B. organische Verbindungen oder Graphit zugesetzt, die beim späteren Erhitzen verbrennen, z.B. um die Verformbarkeit der pastosen Masse zu verbessern oder die Härte und Porosität des fertigen Katalysators günstig zu beeinflussen.

Nach inniger Durchmischung z.B. einer trockenen Mischung der Bestandteile oder eines daraus hergestellten Sprühpulvers werden, gegebenenfalls unter Wasserzugabe, Formkörper hergestellt, die dann getrocknet und bei Temperaturen von etwa 300 bis 1100°C calciniert und damit in die fertigen Katalysatoren überführt werden (1, 4, 9). Nach (12) ist auch eine definierte Verbindung, ein Kaliumferrit der Formel K₂Fe₂₂O₃₄, als Katalysator wirksam.

Aus (5) ist ein Verfahren mit zwei Calcinationsschritten besonders bekannt, bei dem der Rohmasse Methylcellulose oder Graphit zugesetzt wird, die bei 250°C bis 600°C ausgebrannt werden, wonach in einem weiteren Schritt bei 700 bis 800°C auch die verbliebenen Carbonate in die entsprechenden Oxide umgewandelt werden.

Die bekannten Katalysatoren sind jedoch nach wie vor weit davon entfernt, ein ideales Ergebnis zu liefern, d.h. sie bilden nach wie vor unerwünschte Nebenprodukte und erscheinen auch hinsichtlich der spezifischen Leistung (mögliche Raum-Zeit-Ausbeute) und ihrer Haltbarkeit unter technischen Bedingungen (mechanische Stabilität) verbesserungsfähig.

Aufgabe der Erfindung ist es daher, ein Verfahren anzugeben, mit dem es gelingt, hinsichtlich Aktivität und Selektivität sowie der mechanischen Stabilität verbesserte Katalysatoren der eingangs bezeichneten Art herzustellen.

Mit dem erfindungsgemäßen Herstellungsverfahren wird u.a. die Aufgabe gelöst, Katalysatoren herzustellen, die sich gegenüber den bekannten Katalysatoren durch höhere Porosität, höhere Aktivität und bessere Selektivität auszeichnen.

Es wurde gefunden, daß die Herstellung von verbesserten, Eisen und Kalium und gegebenenfalls weitere Elemente enthaltenden Katalysatoren, die sich zur Dehydrierung von Kohlenwasserstoffen, insbesondere zur Dehydrierung von Ethylbenzol zu Styrol eignen, dadurch gelingt, daß man eine feinteilige, vorzugsweise wäßrige Mischung einer Eisenverbindung mit einer Kaliumverbindung und gegebenenfalls Verbindungen weiterer Elemente herstellt, die Mischung gegebenenfalls trocknet, in einem ersten Schritt derart erhitzt (calciniert), daß aus kleineren Einzelteilchen gebildete Konglomerate mit einem Durchmesser von 5 bis 50 µm erhalten werden und in einem zweiten Schritt, vorzugsweise nach Formgebung, auf 300 bis 1000°C erhitzt (calciniert).

Eisen, Kalium sowie gegebenenfalls Promotoren werden in Form von Oxiden oder von Substanzen, die sich beim Erhitzen in Oxide umwandeln, miteinander gemischt.

Als Eisenverbindung wird bevorzugt Fe₂O₃ (Hämatit), FeOOH (Goethit), Fe₃O₄ oder eine andere künstliche oder natürlich vorkommende oxidische Eisenverbindung verwendet.

Als Kaliumverbindung wird bevorzugt Kaliumcarbonat, Kaliumhydroxid oder eine andere in der Wärme zersetzbare Kaliumverbindung wie Kaliumoxalat verwendet, man kann auch eine Kaliumverbindung verwenden, die den vorgesehenen Promotor (d.h. als entsprechendes Anion oder als Doppelsalz) enthält.

Als Eisen- bzw. Kaliumverbindung kann ganz oder teilweise eine Kaliumferritverbindung verwendet werden. Als Promotoren kann neben Cer z.B. mindestens eine Verbindung ausgewählt aus Verbindungen des des Al, Ca, Co, Cr, Cs, Ca, Li, Mg, Mo, Na, W, V oder Zn zugesetzt werden. Besonders bevorzugt sind Verbindungen des Ca, Ce, Mg, Mo oder W.

Das Durchmischen der Rohstoffe und die erste Trocknung soll zu möglichst kleinen Partikeln führen, die z.B. einen Durchmesser von 0,1 bis 10 µm erhalten sollen.

Geeignete Verfahren zur Herstellung solch feinteiliger Mischungen sind an sich bekannt und bestehen z.B. in mahlenden und/oder reibenden Techniken; während für Laborversuche Haushaltsgeräte wie Mixer durchaus geeignet sind, werden für technische Zwecke Kneter, Kollergänge oder Kugelmühlen bevorzugt.

Bei dem intensiven Vermischen von Eisen- und Kaliumverbindungen werden in z.T. erheblichem Umfang Kalium-Eisen-Verbindungen gebildet, wie Kaliumferrrite unterschiedlicher Bruttozusammensetzung. Diese bilden i.a. Kristallite der erwähnten Dimension 0,1 bis 10 µm und sollen bei der späteren Calcinierung lediglich zusammensintern, jedoch nicht verschwinden.

Man erhält schließlich eine feinteilige, trockene oder bevorzugt eine wäßrige Mischung, die z.B. einen Feststoffgehalt von 10 bis 80 % aufweist.

Die Trocknung der wäßrigen Mischung (Sprühmaische) vor dem ersten Erhitzen geschieht zweckmäßig durch Sprühtrocknung. Ungeachtet der Temperatur der Trocknerluft, die z.B. 300 bis 600°C betragen kann, werden die entstehenden Teilchen i.a. auf keine höhere Temperatur als etwa 100, kurzfristig auch bis 150°C erhitzt, da das verdampfende Wasser eine höhere Temperatur verhindert. Die Entstehung genügend kleiner Teilchen wird durch die Art und Weise beeinflußt, wie die Sprüheinrichtung betrieben wird. Als Sprüheinrichtung dient vorteilhaft eine rotierende Sprühdüse oder ein Scheibenrad, deren Drehzahl je nach Feststoffgehalt der Sprühmaische z.B. 10 000 bis 30 000 min⁻¹ betragen kann. Die günstigste Drehzahl wird - abhängig vom Feststoffgehalt der Sprühmaische - am besten durch einen Vorversuch ermittelt.

Das erhaltene Sprühpulver soll erfindungsgemäß aus Konglomeraten mit einem Durchmesser von etwa 5 bis 50 µm, bevorzugt 15 bis 35 µm bestehen, die aus den ursprünglich vorhandenen, entsprechend kleineren Teilchen zusammengesetzt sind. Die Art des Zusammenhalts kann man als Sintern oder Verbacken bezeichnen. Es ist wichtig, daß diese Konglomerate bei der nachfolgenden Erhitzung (Calcinierung) und auch bei der daran anschließenden Formgebung nicht zerstört werden. Die Konglomerate werden stabilisiert, wenn die Mischung im ersten Schritt auf 600°C bis 1200°C erhitzt wird, wobei die Dauer der Erhitzung natürlich ebenso eine Rolle spielt. Es hat sich als zweckmäßig erwiesen, im ersten Schritt für die Dauer von bis zu 24 Stunden zu erhitzen.

Es ist erfindungsgemäß besonders vorteilhaft, das Verfahren auf die Herstellung eines Katalysators anzuwenden, der eine Cerverbindung enthält, wobei es besonders empfehlenswert ist, dem vorcalcinierten Katalysator die Cerverbindung ganz oder teilweise erst nach dem ersten Schritt zuzusetzen und dann abermals zu calcinieren. Es scheint, daß es durch diese Maßnahme gelingt, die wirksame Oberfläche zu vergrößern und so die Aktivität zu verbessern.

Hierzu wird der vorcalcinierten Masse gegebenenfalls die fehlende Cerverbindung, evtl. weitere Promotoren, evtl. auch weiteres Eisenoxid und (weitere) Hilfsmittel zugegeben, vorzugsweise naß gemischt und aus der Mischung Formkörper hergestellt, die dann gegebenenfalls getrocknet und bei 300 bis 1000°C abermals calciniert werden. Es ist erfindungsgemäß günstig, die Temperatur des zweiten Calcinierschrittes z.B. im Bereich von 500 bis 900, bevorzugt nicht über 800°C zu halten, wobei die höchste Calciniertemperatur im zweiten Schritt i.a. um mindestens 30° tiefer liegt als die Calciniertemperatur im ersten Schritt. Bei Temperaturen von deutlich über 800 bis 900°C treten - je nach Zeitdauer der Einwirkung - bei Katalysatoren, die Cer enthalten, bereits größere Kristallite auf, die nicht erwünscht sind.

Als Formkörper werden z.B. Tabletten oder Stränge hergestellt, aber es sind auch andere Formkörper möglich, entsprechend dem für den Einsatz des Katalysators vorgesehenen Verfahren, das in der Regel auf die Verwendung bestimmter Formen und Abmessungen des Katalysators angewiesen ist.

Durch das erfindungsgemäße Verfahren kann die Porosität und die innere Oberfläche der Katalysatoren vergrößert werden, ohne daß die mechanische Stabilität leidet.

Durch das erfindungsgemäße Herstellungsverfahren erreicht man u.a. auch, daß das eingelagerte Cer bzw. dessen Verbindungen kleinere Kristallite bilden, womit eine Vergrößerung der effektiven Oberfläche einhergeht. Dies verbessert sowohl die Aktivität wie auch die Selektivität der Katalysatoren bei Dehydrierungsreaktionen.

Zu Veranschaulichung des nächstbekannten Standes der Technik (EP-A-296 285; D5) werden zunächst Vergleichsversuche angestellt; diese sind in der unten wiedergegebenen Tabelle als Versuch 1 bis 3 aufgeführt:

### Vergleichsversuche 1 bis 3

Aus 100 g FeOOH, 19,5 g K₂CO₃, 11,4 g Ce₂(CO₃)₃, 4,2 g CaCO₃ und 4,4 g WO₃ wurde eine pulverförmige Mischung hergestellt. Nach Wasserzugabe wurde durch Kneten eine verstrangbare Masse erzielt, die zu 3 mm dicken und 1 cm langen Vollsträngen verpreßt wurde. Nach dem Trocknen wurden die Stränge bei 300°C getempert und die Vergleichsproben 1 bis 3 dadurch erhalten, daß jeweils ein Drittel der Masse anschließend bei 700, 800 bzw. 900°C calciniert wurde.

### Herstellbeispiel 1

Aus den vorstehend angegebenen Rohstoffen bzw. -mengen wurde nach Zusatz von 418,5 ml Wasser eine Suspension mit einem Feststoffgehalt von 25 Gew.-% erhalten. Die Suspension wurde sprühgetrocknet, indem sie auf einen in einem heißen Luftstrom angeordneten Drehteller aufgegeben wurde, der mit einer Umdrehungszahl von 28000 min⁻¹ betrieben wurde. Je zwei Proben dieses Sprühpulvers wurden bei 700°C, 800°C und 900°C jeweils eine bzw. 24 Stunden im Umluftofen calciniert, d.h. sechs unterschiedlich vorbehandelte Proben hergestellt. Danach wurde jeweils durch Wasserzugabe und Kneten eine verstrangbare Masse hergestellt und zu Strängen verpreßt. Nach Trocknen und Tempern wurden die Stränge bei 700, 800 und 900°C calciniert (erfindungsgemäß; Katalysatoren 4 bis 21).

### Herstellbeispiel 2:

Aus 100 g FeOOH, 19,5 g K₂CO₃, 4,2 g CaCO₃ und 4,4 g WO₃ wurde ein Sprühpulver hergestellt und dieses eine Stunde bei 900°C calciniert. Danach wurden 12,8 g Ce₂(CO₃)₃ zugegeben und nach Suspendieren in Wasser nochmals versprüht. Aus dem erhaltenen Sprühpulver wurde unter Wasserzugabe und Kneten eine verstrangbare Masse hergestellt und zu Strängen verpreßt. Nach dem Trocknen und Tempern wurden die Stränge bei 700, 800 und 900°C calciniert (erfindungsgemäß; Katalysatoren 22 bis 24).

### Herstellbeispiel 3:

Aus 100 g FeOOH und 12 g K₂CO₂ wurde ein Sprühpulver hergestellt und 24 h bei 900°C calciniert. Nach dem Calcinieren wurden 7,5 g K₂CO₃, 4,2 g CaCO₃, 4,4 g WO₃ und 12,8 g Ce₂(CO₃)₃ zugegeben und nach Suspendieren in Wasser erneut versprüht. Danach wurde unter Wasserzugabe und Kneten eine verstrangbare Masse hergestellt und zu Strängen verpreßt. Nach dem Trocknen und Tempern wurden die Stränge bei 700, 800 und 900°C calciniert (erfindungsgemäß; Katalysatoren 25 bis 27).

### Leistungsuntersuchung

Die Katalysatoren werden in einer Versuchseinrichtung getestet, die dem technisch betriebenen isothermen Verfahren nachgebildet ist. Dazu werden 407 cm³ Vollstrang-Katalysator in ein Reaktionsrohr mit 30 mm Innendurchmesser gefüllt. Zunächst wird während 10 Tagen eine stündliche Menge von 268 g Ethylbenzol und 536 g Wasser in Dampfform übergeleitet, wobei die Temperatur so gewählt wird, daß sich ein Umsatz von 70% einstellt. Danach wird die Zulaufmenge auf 300 g Ethylbenzol und 418 g Wasser geändert. Die Temperatur wird für 3 Tage auf 590°C eingestellt und nach 3 Tagen Umsatz, Selektivität und Zusammensetzung des Reaktionsgemisches ermittelt, indem die Reaktionsprodukte (Flüssigkeit und Abgas) untersucht werden.

**Tabelle 2**

| Katalytische Eigenschaften | | | | | | | | | |
|---|---|---|---|---|---|---|---|---|---|
| Nr. | 2 | 5 | 8 | 11 | 14 | 17 | 20 | 23 | 25 |
| Umsatz | 39,3 | 42,0 | 44,2 | 45,40 | 45,6 | 43,8 | 42,6 | 45,2 | 44,1 |
| Sel.* | 97,9 | 97,8 | 98,0 | 97,7 | 97,9 | 98,1 | 98,0 | 98,0 | 98,1 |

| | | | | | | | | | |
|---|---|---|---|---|---|---|---|---|---|
| * Sel. = Selektivität | | | | | | | | | |

Der Vergleich der physikalischen Eigenschaften der erfindungsgemäßen Katalysatoren 4 bis 27 mit denen der Vergleichsproben 1 bis 3 zeigt, daß die Porosität und die BET-Oberfläche stark gesteigert werden konnten. Der Wirkungsvergleich (Tabelle) zeigt, daß sich Aktivität und Selektivität stark verbessert haben. Gegenüber dem nach dem bisher üblichen Verfahren hergestellten Vergleichskatalysator 2 zeigt beispielsweise Katalysator 25 eine gleichzeitige Verbesserung von Aktivität und Selektivität über den gesamten Umsatzbereich. Dabei verbessert sich der Umsatz um 4,8 %, während sich die Selektivität bei gleicher Temperatur um 0,2 % verbessert. Bezogen auf den gleichen Umsatz von 44 % bedeutet dies eine Selektivitätsverbesserung um 0,35 %.

## Patentansprüche

1. Verfahren zur Herstellung von Eisen und Kalium und gegebenenfalls weitere Elemente enthaltenden Katalysatoren, die sich zur Dehydrierung von Kohlenwasserstoffen zu den entsprechenden olefinisch ungesättigten Kohlenwasserstoffen eignen, **dadurch gekennzeichnet, daß** man eine feinteilige trockene oder wäßrige Mischung einer Eisenverbindung mit einer Kaliumverbindung und gegebenenfalls Verbindungen weiterer Elemente herstellt, die Mischung, falls sie wäßrig ist, trocknet, in einem ersten Schritt derart erhitzt, daß aus kleineren Einzelteilchen gebildete Konglomerate mit einem Durchmesser von 5 bis 50 µm erhalten werden und in einem zweiten Schritt, vorzugsweise nach Formgebung, auf 300 bis 1000°C erhitzt.

2. Verfahren nach Anspruch 1, **dadurch gekennzeichnet, daß** eine wäßrige Mischung mit einem Feststoffgehalt von 10 bis 80 Gewichtsprozent hergestellt und sprühgetrocknet wird.

3. Verfahren nach Anspruch 1, **dadurch gekennzeichnet, daß** die Mischung im ersten Schritt auf 600°C bis 1200°C erhitzt wird.

4. Verfahren nach Anspruch 1, **dadurch gekennzeichnet, daß** als weiteres Element Cer eingesetzt wird.

5. Verfahren nach Anspruch 1, **dadurch gekennzeichnet, daß** die Cerverbindung erst nach dem ersten bzw. vor dem zweiten Erhitzen zugesetzt wird.

6. Verfahren nach Anspruch 1, **dadurch gekennzeichnet, daß** die höchste Temperatur im zweiten Schritt um mindestens 30° tiefer liegt als die Temperatur im ersten Schritt.

7. Verfahren nach einem der Ansprüche 1 oder 2, **dadurch gekennzeichnet, daß** die Temperatur des zweiten Schrittes im Bereich von 500 bis 800°C liegt.

8. Verfahren nach Anspruch 1 **dadurch gekennzeichnet, daß** als Eisenverbindung ein Eisenoxid eingesetzt wird.

9. Verfahren nach Anspruch 1 **dadurch gekennzeichnet, daß** als Eisenverbindung Fe₂O₃, FeOOH oder Fe₃O₄ eingesetzt wird.

10. Verfahren nach Anspruch 1 **dadurch gekennzeichnet, daß** als Kaliumverbindung K₂CO₃, KOH oder eine in der Wärme zu einem Oxid zersetzbare Kaliumverbindung wie Kaliumoxalat eingesetzt wird.

11. Verfahren nach Anspruch 1, **dadurch gekennzeichnet, daß** als Eisen- bzw. Kaliumverbindung ganz oder teilweise eine Kaliumferritverbindung eingesetzt wird.

12. Verfahren nach Anspruch 1, **dadurch gekennzeichnet, daß** zusätzlich mindestens eine Verbindung, ausgewählt aus Verbindungen des Al, Ca, Ce, Co, Cr, Cs, Ca, Li, Mg, Mo, Na, W, V oder Zn eingesetzt wird.

13. Verfahren nach Anspruch 12, **dadurch gekennzeichnet, daß** eine Verbindung, ausgewählt aus Verbindungen des Ca, Ce, Mg, Mo oder W eingesetzt wird.

14. Verfahren zur Dehydrierung alkylaromatischer oder aliphatischer Kohlenwasserstoffe zu entsprechenden Alkenen insbesondere von Ethylbenzol zu Styrol, **dadurch gekennzeichnet, daß** man einen nach dem Verfahren eines der Ansprüche 1 bis 9 erhaltenen Katalysator verwendet.

15. Verfahren nach Anspruch 14, **dadurch gekennzeichnet, daß** als alkylaromatischer Kohlenwasserstoff Ethylbenzol eingesetzt und zu Styrol dehydriert wird.

16. Sprühgetrocknete Mischung, hergestellt nach dem Verfahren gemäß Anspruch 2, die eine innere Oberfläche, bestimmt nach der BET-Methode, von mindestens 3 qm/g aufweist.

## Claims

1. A process for preparing catalysts comprising iron and potassium and, if desired, further elements, which catalysts are suitable for dehydrogenating hydrocarbons to give the corresponding olefinically unsaturated hydrocarbons, wherein a finely divided dry or aqueous mixture of an iron compound with a potassium compound and, if desired, compounds of further elements is prepared, the mixture is dried if it is aqueous, heated in a first step so that agglomerates having a diameter of from 5 to 50 µm and formed from smaller individual particles are obtained and, in a second step, preferably after shaping, heated at from 300 to 1000°C.

2. A process as claimed in claim 1, wherein an aqueous mixture having a solids content of from 10 to 80 percent by weight is prepared and spray dried.

3. A process as claimed in claim 1, wherein the mixture is heated at from 600°C to 1200°C in the first step.

4. A process as claimed in claim 1, wherein cerium is used as further element.

5. A process as claimed in claim 1, wherein the cerium compound is added after the first calcination and before the second calcination.

6. A process as claimed in claim 1, wherein the maximum temperature in the second step is at least 30° lower than the temperature in the first step.

7. A process as claimed in claim 1 or 2, wherein the temperature of the second step is in the range from 500 to 800°C.

8. A process as claimed in claim 1, wherein the iron compound used is an iron oxide.

9. A process as claimed in claim 1, wherein the iron compound used is Fe₂O₃, FeOOH or Fe₃O₄.

10. A process as claimed in claim 1, wherein the potassium compound used is K₂CO₃, KOH or a potassium compound such as potassium oxalate which can be decomposed by heat to give an oxide.

11. A process as claimed in claim 1, wherein the iron and potassium compounds used are, completely or in part, a potassium ferrite compound.

12. A process as claimed in claim 1, wherein additional use is made of at least one compound selected from among compounds of Al, Ca, Ce, Co, Cr, Cs, Ca, Li, Mg, Mo, Na, W, V or Zn.

13. A process as claimed in claim 12, wherein a compound selected from among compounds of Ca, Ce, Mg, Mo or W is used.

14. A process for dehydrogenating alkylaromatic or aliphatic hydrocarbons to give the corresponding alkenes, in particular ethylbenzene to give styrene, wherein a catalyst obtained by the process of any of claims 1 to 9 is used.

15. A process as claimed in claim 14, wherein the alkylaromatic hydrocarbon used is ethylbenzene and is dehydrogenated to give styrene.

16. A spray-dried mixture prepared by the process as claimed in claim 2 and having an internal surface area, determined by the BET method, of at least 3 m²/g.

## Revendications

1. Procédé de préparation de catalyseurs contenant du fer et du potassium et éventuellement d'autres éléments, qui conviennent à la déshydrogénation d'hydrocarbures en hydrocarbures à insaturation oléfinique correspondants, **caractérisé en ce que** l'on prépare un mélange sec ou aqueux finement divisé d'un composé du fer avec un composé du potassium et éventuellement des composés d'autres éléments, on le sèche si le mélange est aqueux, on le chauffe dans une première étape de sorte à obtenir des conglomérats formés de petites particules individuelles ayant un diamètre de 5 à 50 µm puis on le chauffe dans une deuxième étape de préférence après un façonnage à une température de 300 à 1 000°C.

2. Procédé selon la revendication 1, **caractérisé en ce qu'**un mélange aqueux avec une teneur en solides de 10 à 80% en poids est préparé et séché par pulvérisation.

3. Procédé selon la revendication 1, **caractérisé en ce que** le mélange est chauffé dans la première étape à une température de 600 à 1 200°C.

4. Procédé selon la revendication 1, **caractérisé en ce que** le cérium est mis en oeuvre en tant qu'autre élément.

5. Procédé selon la revendication 1, **caractérisé en ce que** le composé du cérium est ajouté tout de suite après le premier échauffement ou avant le deuxième échauffement.

6. Procédé selon la revendication 1, **caractérisé en ce que** la température la plus élevée dans la deuxième étape est inférieure d'au moins 30° à la température dans la première étape.

7. Procédé selon l'une quelconque des revendications 1 ou 2, **caractérisé en ce que** la température de la deuxième étape est dans la plage de 500 à 800°C.

8. Procédé selon la revendication 1, **caractérisé en ce qu'**un oxyde de fer est mis en oeuvre en tant que composé du fer.

9. Procédé selon la revendication 1, **caractérisé en ce que** Fe₂O₃, FeOOH ou Fe₃O₄ sont mis en oeuvre en tant que composé du fer.

10. Procédé selon la revendication 1, **caractérisé en ce que** K₂CO₃, KOH ou un composé du potassium thermiquement décomposable en un oxyde, tel que l'oxalate de potassium, sont mis en oeuvre en tant que composé du potassium.

11. Procédé selon la revendication 1, **caractérisé en ce qu'**un composé ferrite de potassium est mis en oeuvre totalement ou partiellement en tant que composé du fer ou du potassium.

12. Procédé selon la revendication 1, **caractérisé en ce que**, de plus, au moins un composé choisi parmi les composés du Al, Ca, Ce, Co, Cr, Cs, Ca, Li, Mg, Mo, Na, W, V ou Zn est mis en oeuvre.

13. Procédé selon la revendication 12, **caractérisé en ce qu'**un composé choisi parmi les composés du Ca, Ce, Mg, Mo ou W, est mis en oeuvre.

14. Procédé de déshydrogénation d'hydrocarbures alkylaromatiques ou aliphatiques en les alcènes correspondants, en particulier de l'éthylbenzène en styrène, **caractérisé en ce que** l'on emploie un catalyseur obtenu selon l'une quelconque des revendications 1 à 9.

15. Procédé selon la revendication 14, **caractérisé en ce que**, l'éthylbenzène en tant qu'hydrocarbure alkylaromatique, est mis en oeuvre et déshydrogéné en styrène.

16. Mélange séché par pulvérisation, préparé d'après le procédé selon la revendication 2, qui présente une surface spécifique interne d'au moins 3 m²/g, déterminée d'après le procédé BET.
